# EUROPEAN PATENT APPLICATION

(11) **EP 4 282 859 A1**
(43) Date of publication of application: **29.11.2023**
(21) Application number: 22175292.6
(22) Date of filing: 25.05.2022
(51) Int. Cl.: C07D 251/70, C08K 5/3492, C09D 167/00

(54) **TRIAZINE CARBAMATE CROSSLINKER**

(71) Applicant: Allnex USA Inc., Alpharetta GA 30009 (US)
(72) Inventor: MAHAJAN, Lalit, Alpharetta, 30009 (US); FROEHLICH, Gerd, Alpharetta, 30009 (US); FLOOD, LAWRENCE, Woodstock, 30189 (US); GARIEPY, Kimberly, Cumming, 30040 (US)
(74) Representative: Gabriel, Kiroubagaranne

(57) **Abstract**

A crosslinker obtainable by reacting one or more compounds of general formula (II) 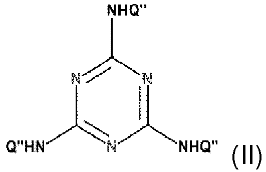 with one or more compounds of general formula (III)

HO-[X-]ᵢR¹ (III),

wherein the crosslinker comprises a mixture of compounds of general formula (I) 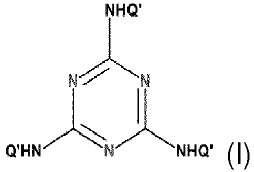 wherein Q' is a radical of formula COO-[X-]ᵢR¹,
wherein each i is independently selected from integers between 1 and 50 for 35 mol% or higher of Q' within the mixture, and i is equal to 0 for the remaining Q' within the mixture,
wherein each X is independently selected from the group consisting of CH₂-CH₂-O, CH₂-CH(CH₃)-O, CH(CH₃)-CH₂-O-, CH₂-C(CH₃)₂-O-, CH₂-CHVin-O, CHVin-CH₂-O, CH₂-CHPh-O-, and CHPh-CH₂-O, in which Ph is phenyl and Vin is vinyl, and
wherein each R¹ is independently selected from the group consisting of C₁-C₃₀ hydrocarbyls.

## Description

### Technical Field

The present invention relates to triazine carbamates crosslinkers, in particular for use in coating compositions, and more in particular for use in waterborne formulations for forming a coating.

### Background

Tris(alkoxycarbonylamino) triazines are commercially available crosslinking agents. They typically comprise in their chemical structure a combination of various alkyl carbamate functionalities. They are typically supplied in an organic solvent such as n-butanol as they are not water-soluble. It is therefore difficult to use them in waterborne coating formulations. An interesting feature of such compounds is that they do not contain or emit formaldehyde on cure. Their human toxicity is, therefore, lower. They are also particularly unsuitable for self-crosslinking reactions, i.e., they do not form a film in absence of other reactive compounds. When used to crosslink reactive compounds such as hydroxy- and epoxy-containing polymers, they give highly durable, acid-resistant films that exhibit a favorable balance of hardness and flexibility. Such films typically find applications in automotive topcoats, exterior can varnishes, and coil coatings. Tris(alkoxycarbonylamino) triazines can be employed as the sole crosslinker in a coating or ink formulation, or it may be used at lower levels in combination with other crosslinkers, such as amino resins or isocyanates, in order to obtain a balance of properties.

Aside from Tris(alkoxycarbonylamino) triazines, a few other available non-formaldehyde crosslinkers providing carbamate functionality are available. Examples of such alternatives are isocyanates and blocked isocyanates. These can provide excellent properties but generate hazardous blocking agents and free isocyanates.

### Summary

It is hence an object of the present invention to provide a carbamate crosslinker that overcomes at least partially some of the above drawbacks.

More particularly, it is an object of the present invention to provide a stable water-compatible carbamate crosslinker enabling high crosslinking density, and associated with low environmental or toxicity issues.

The crosslinker of the first aspect, the mixture of the second aspect, and the compound of the third aspect may have one or more of the following advantages:
- They may be self-crosslinkable. This means that they may be able to react with themselves to form a crosslinked product (typically a film). No further reactive compounds, other than the crosslinker, mixture, or compound itself, is therefore needed for obtaining a crosslinked product. Hence, films can be typically obtained in presence of only a low amount of further reactive compounds, or even in the absence thereof.
- They may be formaldehyde-free. This means that they do not need to contain formaldehyde and that they typically do not contain formaldehyde. Also, they do not emit formaldehyde on cure. Their human toxicity is, therefore, low.
- They are particularly well-suited to form low-VOC (Volatile Organic Compound) formulations as they tend to be dispersible in water.
- They may allow the formation of coatings having high crosslinking density.
- They may be compatible with waterborne acrylic, polyester and epoxy binders.

The aqueous dispersion of the fourth aspect may have one or more of the following advantages:
- It may be stable.
- It may be very low in VOC.
- It may allow the formation of coatings having excellent solvent resistance on multiple substrates, including metal, glass, and paper.

In a first aspect, the present invention relates to a crosslinker obtainable by reacting one or more compounds of general formula (II) 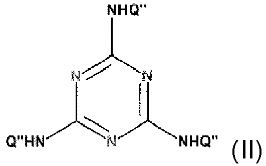 with one or more compounds of general formula (III)

HO-[X -]ᵢR¹ (III),

wherein each Q" is a radical of formula COOR²,
wherein each R² is independently selected from the group consisting of C₁-C₃₀ hydrocarbyls,
wherein each X is independently selected from the group consisting of CH₂-CH₂-O, CH₂-CH(CH₃)-O, CH(CH₃)-CH₂-O-, CH₂-C(CH₃)₂-O-, CH₂-CHVin-O, CHVin-CH₂-O, CH₂-CHPh-O-, and CHPh-CH₂-O, in which Ph is phenyl and Vin is vinyl,
wherein each i is independently selected from integers between 1 and 50, wherein each R¹ is independently selected from the group consisting of C₁-C₃₀ hydrocarbyls,
wherein the crosslinker comprises a mixture of compounds of general formula (I) 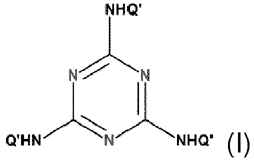
wherein Q' is a radical of formula COO-[X-]ᵢR¹,
wherein each i is independently selected from integers between 1 and 50 for 35 mol% or higher of Q' within the mixture, and i is equal to 0 for the remaining Q' within the mixture,
wherein each X is independently selected from the group consisting of CH₂-CH₂-O, CH₂-CH(CH₃)-O, CH(CH₃)-CH₂-O-, CH₂-C(CH₃)₂-O-, CH₂-CHVin-O, CHVin-CH₂-O, CH₂-CHPh-O-, and CHPh-CH₂-O, in which Ph is phenyl and Vin is vinyl, and
wherein each R¹ is independently selected from the group consisting of C₁-C₃₀ hydrocarbyls.

In a second aspect, the present invention relates to a mixture of compounds of general formula (I) 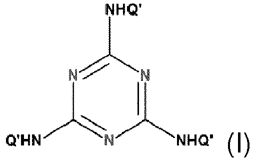
wherein Q' is a radical of formula COO-[X-]ᵢR¹,
wherein each i is independently selected from integers between 1 and 50 for 35 mol% or higher of Q' within the mixture, and i is equal to 0 for the remaining Q' within the mixture,
wherein each X is independently selected from the group consisting of CH₂-CH₂-O, CH₂-CH(CH₃)-O, CH(CH₃)-CH₂-O-, CH₂-C(CH₃)₂-O-, CH₂-CHVin-O, CHVin-CH₂ -O, CH₂-CHPh-O-, and CHPh-CH₂-O, in which Ph is phenyl and Vin is vinyl, and
wherein each R¹ is independently selected from the group consisting of C₁-C₃₀ hydrocarbyls.

In a third aspect, the present invention relates to a compound of general formula (I) 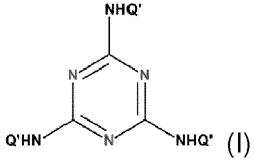 selected from the list consisting of compounds a) wherein one of the Q' groups is a radical of formula COOCH₂CH₂CH₂CH₃, and wherein two of the Q' groups are radicals of formula COO-[CH₂-CH₂-O-]₂CH₃, b) wherein one of the Q' groups is a radical of formula COOCH₂CH₂CH₂CH₃, and wherein two of the Q' groups are radicals of formula COO-[CH₂-CH₂-O-]₂CH₂CH₂CH₂CH₃, c) wherein one of the Q' groups is a radical of formula COOCH₂CH₂CH₂CH₃, and wherein two of the Q' groups are radicals of formula COO-[CH₂-CH(CH₃)-O-]₂CH₃, and d) wherein one of the Q' groups is a radical of formula COOCH₂CH₂CH₂CH₃, and wherein two of the Q' groups are radicals of formula COOCH₂CH₂OCH₂CH₂CH₂CH₃.

In a fourth aspect, the present invention relates to a waterborne (coating) composition comprising one of a crosslinker according to any embodiment of the first aspect, a mixture according to any embodiment of the second aspect, or a compound according to any embodiment the third aspect.

In a fifth aspect, the present invention relates to a method for forming a crosslinker according to the first aspect, a mixture according to the second aspect, or a compound according to the third aspect, comprising the steps of reacting one or more compounds of general formula (II) 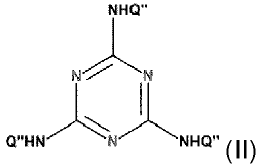 with one or more compounds of general formula (III)

HO-[X-]ᵢR¹ (III),

in absence of a catalyst,
wherein Q" is a radical of formula COOR²,
wherein each R² is independently selected from the group consisting of C₁-C₃₀ hydrocarbyls,
wherein each X is independently selected from the group consisting of CH₂-CH₂-O, CH₂-CH(CH₃)-O, CH(CH₃)-CH₂-O-, CH₂-C(CH₃)₂-O-, CH₂-CHVin-O, CHVin-CH₂-O, CH₂-CHPh-O-, and CHPh-CH₂-O, in which Ph is phenyl and Vin is vinyl,
wherein each i is independently selected from integers between 1 and 50, and wherein each R¹ is independently selected from the group consisting of C₁-C₃₀ hydrocarbyls.

In a sixth aspect, the present invention relates to a method for forming a coating comprising reacting a crosslinker according to any embodiment of the first aspect, a mixture according to any embodiment of the second aspect, or a compound according to any embodiment of the third aspect, with itself, with the proviso that the reaction is performed in presence of at most 30 wt% with respect to the amount of compounds of general formula (I), of further compounds, other than compounds comprising a 1, 3, 5-triazine carbamate moiety, capable of condensing with compounds of general formula (I).

In a seventh aspect, the present invention relates to a coating comprising a crosslinked crosslinker according to any embodiment of the first aspect, a crosslinked mixture according to any embodiment of the second aspect, or a crosslinked compound according to any embodiment of the third aspect, with the proviso that no further compounds, other than compounds comprising a 1, 3, 5-triazine carbamate moiety, are condensed with compounds of general formula (I), or that at most 30 wt% with respect to the amount of compounds of general formula (I), of further compounds, other than compounds comprising a 1, 3, 5-triazine carbamate moiety, are condensed with compounds of general formula (I). In an eighth aspect, the present invention relates to a method for co-crosslinking a crosslinker according to any embodiment of the first aspect, a mixture according to any embodiment of the second aspect, or a compound according to any embodiment of the third aspect, with a further compound, wherein the method comprises a step of heating up the mixture and the further compound at a temperature of from 100 to 150°C.

### Detailed description

As used herein and unless provided otherwise, the term "waterborne composition" is meant to designate water-based solutions, water-based dispersions and water-based emulsions. As typically known in the technical field, the term "water-based solution" is meant to refer to compounds, and typically oligomer and/or polymer particles, being mixed with an aqueous medium in a continuous phase. The term "water-based dispersion" is meant to refer to a mixture in which the compounds are dispersed as insoluble particles in the aqueous medium. The term "water-based emulsion" is meant to refer to a mixture in which the compounds are suspended as liquid droplets throughout the aqueous medium.

In the first aspect, the present invention relates to a crosslinker obtainable by reacting one or more compounds of general formula (II) 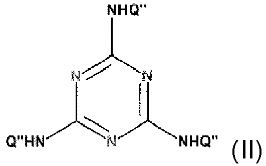 with one or more compounds of general formula (III)

HO-[X -]ᵢR¹ (III),

wherein each Q" is a radical of formula COOR²,
wherein each R² is independently selected from the group consisting of C₁-C₃₀ hydrocarbyls,
wherein each X is independently selected from the group consisting of CH₂-CH₂-O, CH₂-CH(CH₃)-O, CH(CH₃)-CH₂-O-, CH₂-C(CH₃)₂-O-, CH₂-CHVin-O, CHVin-CH₂-O, CH₂-CHPh-O-, and CHPh-CH₂-O, in which Ph is phenyl and Vin is vinyl,
wherein each i is independently selected from integers between 1 and 50,
wherein each R¹ is independently selected from the group consisting of C₁-C₃₀ hydrocarbyls,
wherein the crosslinker comprises a mixture of compounds of general formula (I) 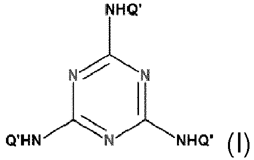
wherein Q' is a radical of formula COO-[X-]ᵢR¹,
wherein each i is independently selected from integers between 1 and 50 for 35 mol% or higher of Q' within the mixture, and i is equal to 0 for the remaining Q' within the mixture,
wherein each X is independently selected from the group consisting of CH₂-CH₂-O, CH₂-CH(CH₃)-O, CH(CH₃)-CH₂-O-, CH₂-C(CH₃)₂-O-, CH₂-CHVin-O, CHVin-CH₂-O, CH₂-CHPh-O-, and CHPh-CH₂-O, in which Ph is phenyl and Vin is vinyl, and
wherein each R¹ is independently selected from the group consisting of C₁-C₃₀ hydrocarbyls.

The crosslinker according to the present invention is obtainable by a trans-carbamoylation reaction wherein radicals of formula [X -]ᵢR¹ come to replace hydrocarbyl chains R² in formula (II) to give formula (I). Compound (II) for use herein may be a pure compound or a mixture of compounds falling under the general formula (II). Compounds of general formula (II) are most readily available as a mixture of compounds falling under general formula (II) and such a mixture of compounds is an acceptable starting material for preparing a crosslinker, a mixture, or a compound according to the present invention.

Formula (II) can also be depicted in its expended form:

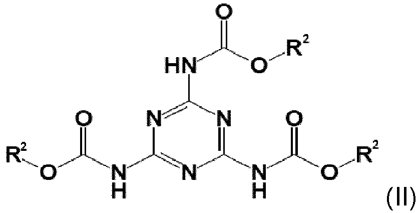

Compounds of general formula (II) are called tris(alkoxycarbonylamino)triazines (also sometimes referred to as TACT).

R² is independently selected from the group consisting of C₁-C₃₀ hydrocarbyls, preferably from the group consisting of C₁-C₁₅ hydrocarbyls, more preferably from the group consisting of C₁-C₁₂ hydrocarbyls, yet more preferably from the group consisting of C₁-C₈ hydrocarbyls, still more preferably from the group consisting of C₁-C₆ hydrocarbyls, yet still more preferably from the group consisting of C₁-C₄ hydrocarbyls, yet still more preferably from the group consisting of C₁ and C₄ (e.g. n-butyl) hydrocarbyls. Linear hydrocarbyl chains are preferred for R². It is an advantage of crosslinkers according to the first aspect obtained from compounds (II) wherein R² is a relatively shorter hydrocarbyl chain that they are expected to be more water-compatible than the crosslinkers according to the first aspect obtained from compounds (II) wherein R² is a relatively longer hydrocarbyl chain. Nevertheless, even for the longer R² chains, water compatibility will be improved with respect to the starting material.

An example of one or more compounds of general formula (II) is a mixture of compounds of general formula (II) comprising compounds where all groups R² are methyl, compounds where all groups R² are n-butyl, and compounds where some groups R² are methyl and the other groups R² are n-butyl. A particular example of such a mixture is CYMEL^{®} NF 2000, commercially available from allnex USA, Inc.

It has been observed that the exact nature of the radical [X -]ᵢR¹ present in compound (I) can be varied within the defined range while keeping its advantageous properties. It is, therefore, acceptable to use more than one different compound (III) for the preparation of compound (I). It is simpler, however, to use a single compound (I).

The various possible X share a hydrophilic nature and length.

In embodiments, each X may be independently selected from the group consisting of -CH₂-CH₂-O, and -CH₂-CH(CH₃)-O.

The nature of X and its proportions in case of mixtures are typically the same for the one or more compounds of general formula (III) and for the mixture of compounds comprised in the crosslinker (I).

i and its proportions in case of mixtures are typically the same for the one or more compounds of general formula (III) and for the mixture of compounds (I) comprised in the crosslinker as far as i different from zero are concerned.

In embodiments, each i different from zero may be independently selected from integers between 1 and 50, preferably between 1 and 30, more preferably between 1 and 15, yet more preferably between 1 and 10, even more preferably between 1 and 5, yet even more preferably between 2 and 3, and most preferably each i different from zero is 2.

The nature of R¹ and its proportions in case of mixtures is typically the same for the one or more compounds of general formula (III) and for the mixture of compounds (I) comprised in the crosslinker as far as i different from zero are concerned.

The nature of R¹ and its proportions in case of mixtures is typically the same as the nature of R² and its proportions for the one or more compounds of general formula (III) and for the mixture of compounds (II) comprised in the crosslinker as far as i equal to zero is concerned.

R¹ is independently selected from the group consisting of C₁-C₃₀ hydrocarbyls. In embodiments, R¹ can be an alkyl, linear or branched, comprising or not a cyclic moiety, an aryl, an alkylaryl, or an arylalkyl. Linear alkyls are preferred.

In embodiments, R¹ may be independently selected from the group consisting of C₁-C₂₀ hydrocarbyls, preferably C₁-C₁₀ hydrocarbyls, more preferably C₁-C₆ hydrocarbyls, and most preferably C₁-C₄ hydrocarbyls. Shorter hydrocarbyls are preferred in view of their better water compatibility.

Examples of one or more compounds of general formula (III) are 2-(2-methoxyethoxy)ethanol, 2-(2-butoxyethoxy)ethanol, dipropylene glycol monomethyl ether, and 2-Butoxyethanol.

Typically, the crosslinker of the present invention is free of formaldehyde.

Typically, the mixture of compounds comprised in the crosslinker is also free of formaldehyde.

The crosslinker according to the first aspect and the mixture of compounds comprised therein comprises compounds obtainable by reacting one or more compounds of general formula (II) with one or more compounds of general formula (III) but may also comprise unreacted starting materials that might remain in the reaction media at the end of the reaction (e.g., compounds of general formula (I) wherein each Q' has i equal to zero). In embodiments, at least 70 mol%, preferably at least 80 mol%, more preferably at least 90 mol%, even more preferably at least 95 mol%, and more preferably substantially all of the compounds of the crosslinker may be according to general formula (I). The crosslinker may be in a liquid media, i.e., it may be in contact with a solvent. For instance, it can be suspended, dispersed, or dissolved in a solvent. The solvent preferably comprises water and the crosslinker is, in that case, comprised in an aqueous dispersion. This is the subject of the fourth aspect.

In embodiments, at least 70 mol%, preferably at least 80 mol%, more preferably at least 90 mol%, more preferably at least 95 mol%, yet more preferably at least 99 mol%, and most preferably substantially all of the compounds of the mixture of compounds of general formula (I) may have at least one Q' radical having an i different from 0. In other words, in these embodiments, at most 30 mol%, preferably at most 20 mol%, more preferably at most 10 mol%, more preferably at most 5 mol%, yet more preferably at most 1 mol%, and most preferably substantially none of the compounds of the mixture of compounds of general formula (I) have all their Q' radicals equal to zero. These compounds of general formula (I) which have all their Q' radicals equal to zero are typically unreacted compounds of general formula (II).

In embodiments, each X may be independently selected from the group consisting of -CH₂-CH₂- O, and -CH₂-CH(CH₃)-O.

In embodiments, each i may be independently selected from integers between 1 and 50, preferably between 1 and 30, more preferably between 1 and 15, yet more preferably between 1 and 10, even more preferably between 1 and 5, yet even more preferably between 2 and 3, for 35 mol% or higher of Q' within the mixture, and i is equal to 0 for the remaining Q' within the mixture.

In preferred embodiments, each i may be independently selected from integers between 1 and 50, preferably between 1 and 30, more preferably between 1 and 15, yet more preferably between 1 and 10, even more preferably between 1 and 5, yet even more preferably between 2 and 3, for 50 to 80 mol% of Q' within the mixture, and i is equal to 0 for the remaining Q' within the mixture.

In more preferred embodiments, each i may be independently selected from integers between 1 and 50, preferably between 1 and 30, more preferably between 1 and 15, yet more preferably between 1 and 10, even more preferably between 1 and 5, yet even more preferably between 2 and 3, for 57 to 77 mol% of Q' within the mixture, and i is equal to 0 for the remaining Q' within the mixture.

In even more preferred embodiments, each i may be independently selected from integers between 1 and 50, preferably between 1 and 30, more preferably between 1 and 15, yet more preferably between 1 and 10, even more preferably between 1 and 5, yet even more preferably between 2 and 3, for 59 to 77 mol% of Q' within the mixture, and i is equal to 0 for the remaining Q' within the mixture.

In yet more preferred embodiments, i may equal 1 or 2 for from 59 to 77 mol% of Q' within the mixture, and i is equal to 0 for the remaining Q' within the mixture.

In most preferred embodiments, i may equal 2 for from 59 to 77 mol% of Q' within the mixture, and i is equal to 0 for the remaining Q' within the mixture.

In embodiments, at least 5 mol%, preferably at least 10 mol%, more preferably at least 15 mol%, yet more preferably at least 20 mol% of the compounds of formula (I) in the mixture have an i different from 0, e.g. selected from integers between 1 and 50, for all its three Q' groups.

In embodiments, from 5 mol% to 40 mol%, preferably 10 to 32 mol%, more preferably 16 to 26 mol%, yet more preferably 19 to 23 mol%, and most preferably from 20 to 22 mol% of the compounds of formula (I) in the mixture have an i different from 0, e.g., selected from integers between 1 and 50, for all its three Q' groups.

In embodiments, from 5 mol% to 40 mol%, preferably 10 to 32 mol%, more preferably 16 to 26 mol%, yet more preferably 19 to 23 mol%, and most preferably from 20 to 22 mol% of the compounds of formula (I) in the mixture have an i selected from integers between 1 and 30 for all its three Q' groups.

In embodiments, from 5 mol% to 40 mol%, preferably 10 to 32 mol%, more preferably 16 to 26 mol%, yet more preferably 19 to 23 mol%, and most preferably from 20 to 22 mol% of the compounds of formula (I) in the mixture have an i selected from integers between 1 and 15 for all its three Q' groups.

In embodiments, from 5 mol% to 40 mol%, preferably 10 to 32 mol%, more preferably 16 to 26 mol%, yet more preferably 19 to 23 mol%, and most preferably from 20 to 22 mol% of the compounds of formula (I) in the mixture have an i selected from integers between 1 and 10 for all its three Q' groups.

In embodiments, from 5 mol% to 40 mol%, preferably 10 to 32 mol%, more preferably 16 to 26 mol%, yet more preferably 19 to 23 mol%, and most preferably from 20 to 22 mol% of the compounds of formula (I) in the mixture have an i selected from integers between 1 and 5 for all its three Q' groups.

In embodiments, from 5 mol% to 40 mol%, preferably 10 to 32 mol%, more preferably 16 to 26 mol%, yet more preferably 19 to 23 mol%, and most preferably from 20 to 22 mol% of the compounds of formula (I) in the mixture have an i selected from integers between 2 and 3 for all its three Q' groups.

In embodiments, from 5 mol% to 40 mol%, preferably 10 to 32 mol%, more preferably 16 to 26 mol%, yet more preferably 19 to 23 mol%, and most preferably from 20 to 22 mol% of the compounds of formula (I) in the mixture have an i equal to 2 for all its three Q' groups.

Any feature of the first aspect may be as correspondingly described in the fifth aspect.

In the second aspect, the present invention relates to a mixture of compounds of general formula (I) 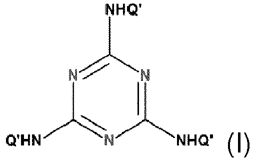
wherein Q' is a radical of formula COO-[X-]ᵢR¹,
wherein each i is independently selected from integers between 1 and 50 for 35 mol% or higher of Q' within the mixture, and i is equal to 0 for the remaining Q' within the mixture,
wherein each X is independently selected from the group consisting of CH₂-CH₂-O, CH₂-CH(CH₃)-O, CH(CH₃)-CH₂-O-, CH₂-C(CH₃)₂-O-, CH₂-CHVin-O, CHVin-CH₂-O, CH₂-CHPh-O-, and CHPh-CH₂-O, in which Ph is phenyl and Vin is vinyl, and
wherein each R¹ is independently selected from the group consisting of C₁-C₃₀ hydrocarbyls.

In embodiments, the composition of the mixture of the second aspect may be the same as the composition of the crosslinker of the first aspect.

Any feature of the second aspect may be as correspondingly described in the first aspect.

In the third aspect, the present invention relates to a compound of general formula (I) 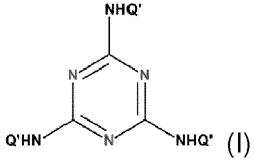 selected from the list consisting of compounds a) wherein one of the Q' groups is a radical of formula COOCH₂CH₂CH₂CH₃, and wherein two of the Q' groups are radicals of formula COO-[CH₂-CH₂-O-]₂CH₃, b) wherein one of the Q' groups is a radical of formula COOCH₂CH₂CH₂CH₃, and wherein two of the Q' groups are radicals of formula COO-[CH₂-CH₂-O-]₂CH₂CH₂CH₂CH₃, c) wherein one of the Q' groups is a radical of formula COOCH₂CH₂CH₂CH₃, and wherein two of the Q' groups are radicals of formula COO-[CH₂-CH(CH₃)-O-]₂CH₃, and d) wherein one of the Q' groups is a radical of formula COOCH₂CH₂CH₂CH₃, and wherein two of the Q' groups are radicals of formula COOCH₂CH₂OCH₂CH₂CH₂CH₃.

These compounds are examples of individual compounds present, in some embodiments, in the crosslinker of the first aspect or in the mixture of the second aspect.

In a first preferred embodiment, the present invention relates to a compound of general formula (I) 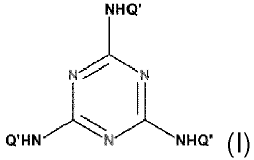 wherein one of the Q' groups is a radical of formula COOCH₂CH₂CH₂CH₃, and wherein two of the Q' groups are radicals of formula COO-[CH₂-CH₂-O-]₂CH₃.

In a second preferred embodiment, the present invention relates to a compound of general formula (I) 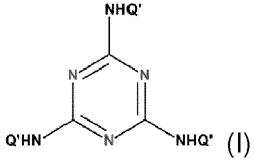 wherein one of the Q' groups is a radical of formula COOCH₂CH₂CH₂CH₃, and wherein two of the Q' groups are radicals of formula

COO-[CH₂-CH₂- O-]₂CH₂CH₂CH₂CH₃.

In a third preferred embodiment, the present invention relates to a compound of general formula (I) 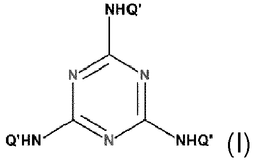 wherein one of the Q' groups is a radical of formula COOCH₂CH₂CH₂CH₃, and wherein two of the Q' groups are radicals of formula COO-[CH₂-CH(CH₃)-O-]₂CH₃.

In a fourth preferred embodiment, the present invention relates to a compound of general formula (I) 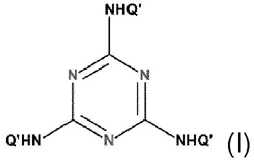 wherein one of the Q' groups is a radical of formula COOCH₂CH₂CH₂CH₃, and wherein two of the Q' groups are radicals of formula COOCH₂CH₂OCH₂CH₂CH₂CH₃.

In the fourth aspect, the present invention relates to a waterborne (coating) composition comprising a crosslinker according to any embodiment of the first aspect, a mixture according to any embodiment of the second aspect, or a compound according to any embodiment of the third aspect.

In embodiments, the waterborne composition may comprise from 5 to 50 wt% water, preferably from 10 to 25 wt% water.

Preferably, the waterborne composition does not comprise any organic solvent. In embodiments, the waterborne composition may comprise from 0 to 10 wt%, preferably from 0 to 4 wt%, of a water-miscible organic solvent. These low amounts of organic solvent make the waterborne composition still qualifying as a low VOC waterborne composition.

Preferably, the sum of the amount of water and the amount of water-miscible organic solvent does not exceed 50 wt%.

In embodiments, the waterborne composition may comprise from 5 to 50 wt% water and from 0 to 50 wt%, preferably 0 to 10 wt%, more preferably 0 to 4 wt%, of a water-miscible organic solvent, wherein the sum of the amount of water and the amount of water-miscible organic solvent does not exceed 50 wt%.

Preferably, the waterborne composition may comprise from 10 to 25 wt% water and from 0 to 10 wt%, preferably 0 to 4 wt% of a water-miscible organic solvent.

When present, any water-miscible organic solvent is suitable. Preferably, the organic solvent may comprise a compound having the following generic formula X= HO-[-X'-]ⱼ-R³

Wherein R³ is independently selected from the group consisting of C₁-C₃₀ hydrocarbyls, and is preferably equal to R¹.

Wherein X' is independently selected from the group consisting of CH₂-CH₂-O, CH₂-CH(CH₃)-O, CH(CH₃)-CH₂-O-, CH₂-C(CH₃)₂-O-, CH₂-CHVin-O, CHVin-CH₂-O, CH₂-CHPh-O-, and CHPh-CH₂-O, in which Ph is phenyl and Vin is vinyl, and is preferably equal to X.

Wherein j is independently selected from integers between 1 and 50 and is preferably equal to i.

In embodiments, the aqueous dispersion may comprise from 50 to 95 wt% of a crosslinker according to any embodiment of the first aspect, of a mixture according to any embodiment of the second aspect, or of a compound according to any embodiment of the third aspect.

In embodiments, the sum of a) the water, b) the optional water-miscible organic solvent, and c) the crosslinker according to any embodiment of the first aspect, the mixture according to any embodiment of the second aspect, or the compound according to any embodiment of the third aspect, amounts to at least 90 wt%, preferably at least 95 wt%, more preferably at least 99 wt%, most preferably substantially all of the water dispersion.

Any feature of the fourth aspect may be as correspondingly described in the first aspect, the second aspect, or the third aspect.

In the fifth aspect, the present invention relates to a method for forming a crosslinker according to the first aspect, a mixture according to the second aspect, or a compound according to the third aspect, comprising the steps of reacting one or more compounds of general formula (II) 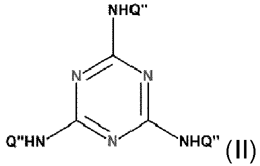 with one or more compounds of general formula (III)

HO-[X-]ᵢR¹ (III),

in absence of a catalyst,
wherein Q" is a radical of formula COOR²,
wherein each R² is independently selected from the group consisting of C₁-C₃₀ hydrocarbyls,
wherein each X is independently selected from the group consisting of CH₂-CH₂-O, CH₂-CH(CH₃)-O, CH(CH₃)-CH₂-O-, CH₂-C(CH₃)₂-O-, CH₂-CHVin-O, CHVin-CH₂-O , CH₂-CHPh-O-, and CHPh-CH₂-O, in which Ph is phenyl and Vin is vinyl,
wherein each i is independently selected from integers between 1 and 50, and wherein each R¹ is independently selected from the group consisting of C₁-C₃₀ hydrocarbyls.

If the one or more compounds of general formula (II) are obtained (e.g. purchased) dissolved in an organic solvent, at least 90 wt%, preferably 95 wt% or more, of this solvent can be beneficially removed prior to adding the one or more compounds of general formula (III) according to techniques well known to those skilled in the art.

Performing this preliminary solvent removal step is particularly beneficial if (III) has a boiling point of 140°C or less.

In embodiments, the molar ratio of compounds of general formula (II) to compounds of general formula (III) may be from 1 : 4.5 to 1 : 1.5. Preferably, this ratio may be from 1 : 4 to 1 : 2, more preferably, this ratio may be from 1 : 3.5 to 1 : 2.5. Most preferably, this ratio is 1 : 3. Such ratios are advantageous because they give the best self-crosslinking performances and give in general very good water and solvent resistance properties after co-crosslinking.

The reaction between (II) and (III) is preferably performed under an inert atmosphere, e.g., in nitrogen, according to techniques well known to those skilled in the art. The reaction temperature is typically comprised in a range from 60 to 180°C, whereas the reaction pressure is typically comprises in a range from 50 to 500 mbar.

No catalyst is needed for the method according to the fifth aspect. The inventors have even noticed that better results are surprisingly obtained when no catalysts are used.

At the end of the reaction, water can be added in order to form a waterborne composition, in particular an aqueous dispersion. Water is preferably added gradually. Water is preferably added until the solid content in the waterborne composition reaches from 75 to 95 wt%.

It is also possible to add, in addition to water, a water-miscible organic solvent to reach a final solid content in the range of 40-94 wt%. An example of suitable water-miscible organic solvent is ethylene glycol monobutyl ether. Adding such a solvent can help reduce the viscosity of the dispersion.

Any feature of the fifth aspect may be as correspondingly described in the first, second, or third aspect.

In a sixth aspect, the present invention relates to a method for forming a coating comprising reacting a crosslinker according to any embodiment of the first aspect, a mixture according to any embodiment of the second aspect, or a compound according to any embodiment of the third aspect, with itself, with the proviso that the reaction is performed in presence of at most 30 wt%, at most 25 wt%, at most 20 wt%, at most 15 wt%, at most 10 wt%, at most 5 wt%, at most 3 wt%, or even at most 1 wt% with respect to the amount of compounds of general formula (I), of further compounds, other than compounds comprising a 1, 3, 5-triazine carbamate moiety, capable of condensing with compounds of general formula (I). This means that if there is 100g of compounds of general formula (I) in the reaction media, no more than 30g, no more than 25g, no more than 20g, no more than 15g, no more than 10g, no more than 5g, or even no more than 1g of compounds, other than compounds comprising a 1, 3, 5-triazine carbamate moiety, capable of condensing with compounds of general formula (I), may be present in the reaction media.

Typically, in the sixth aspect, the present invention relates to a method for forming a coating comprising reacting a crosslinker according to any embodiment of the first aspect, a mixture according to any embodiment of the second aspect, or a compound according to any embodiment of the third aspect, with itself, with the proviso that the reaction is performed in presence of at most 30 wt%, at most 25 wt%, at most 20 wt%, at most 15 wt%, at most 10 wt%, at most 5 wt%, at most 3 wt%, or even at most 1 wt% with respect to the amount of compounds of general formula (I) of further compounds not falling under general formula (I) but capable of condensing with compounds of general formula (I).

Stated alternatively, in the sixth aspect, the present invention relates to the use of a crosslinker according to any embodiment of the first aspect, a mixture according to any embodiment of the second aspect, or a compound according to any embodiment of the third aspect for forming a coating by self-crosslinking.

In embodiments, the coating may be as described hereinafter in the seventh aspect.

The reaction referred to in the sixth aspect is a self-crosslinking reaction, i.e., a crosslinking reaction occurring between a) any of the crosslinker according to any embodiment of the first aspect, the mixture according to any embodiment of the second aspect, or the compound according to any embodiment of the third aspect, and b) itself. The crosslinker according to any embodiment of the first aspect, the mixture according to any embodiment of the second aspect, and the compound according to any embodiment of the third aspect, all have this property of being able to react with themselves, thereby forming a crosslinked network, thereby forming a crosslinked product (typically a film or a coating).

In embodiments, the self-crosslinking reaction is performed in presence of at most 30 wt%, preferably at most 25 wt%, more preferably at most 20 wt%, yet more preferably at most 15 wt%, yet more preferably at most 10 wt%, yet more preferably at most 5 wt%, even more preferably at most 3 wt%, and yet even more preferably at most 1 wt% with respect to the amount of compounds of general formula (I), of further compounds, other than compounds comprising a 1, 3, 5-triazine carbamate moiety, capable of condensing with compounds of general formula (I).

This means that, in these embodiments, up to 30 wt%, up to 25 wt%, up to 20 wt%, up to 15 wt%, up to 10 wt%, up to 5 wt%, up to 3 wt%, or even up to 1 wt% of compounds (other than those comprising a 1, 3, 5-triazine carbamate moiety) not falling under general formula (I) but capable of condensing with crosslinkers, mixtures, or compounds according to general formula (I) are allowed to be present during the reaction. The reason for allowing up to 30 wt% of compounds not falling under general formula (I) is that in presence of such a small amount of other reactive compounds, the reaction remains mostly a self-crosslinking reaction.

Most preferably, the self-crosslinking reaction is performed in absence of further compounds, other than compounds comprising a 1, 3, 5-triazine carbamate moiety, capable of condensing with compounds of general formula (I). This means that, in these embodiments, the only further compounds allowed to be present during the self-crosslinking reaction are compounds comprising a 1, 3, 5-triazine carbamate moiety but that do not fall under the general formula (I). This also means that, in these embodiments, further compounds (other than those comprising a 1, 3, 5-triazine carbamate moiety) capable of condensing with compounds of general formula (I) are not allowed to be present during the reaction. This exception results from the fact that upon formation of crosslinkers according to the first aspect, a small amount of side products comprising a 1, 3, 5-triazine carbamate moiety, but not falling under general formula (I), could be formed. The presence of such side-products typically does not interfere significantly with the properties of the crosslinkers according to the first aspect, mixtures according to the second aspect, or compounds according to the third aspect. Furthermore, removing such side-products is time-consuming, energy-consuming, and wasteful. There are, therefore, typically not removed from the crosslinkers according to the first aspect after their synthesis.

In embodiments, the reaction step may comprise subjecting the crosslinker to a temperature of at least 120°C, or even at least 130°C. For instance, the reaction step may comprise subjecting the crosslinker to a temperature of from 120°C to 190°C.

In embodiments, the further compounds capable of condensing with compounds of general formula (I) may have one or more of the following functionalities: aldehyde, hydroxyl, epoxide, carboxylic, and anhydride.

Any feature of the sixth aspect may be as correspondingly described in the first, second, third, or seventh aspect.

In a seventh aspect, the present invention relates to a coating comprising a crosslinked crosslinker according to any embodiment of the first aspect, a crosslinked mixture according to any embodiment of the second aspect, or a crosslinked compound according to any embodiment of the third aspect, with the proviso that no further compounds, other than compounds comprising a 1, 3, 5-triazine carbamate moiety, are condensed with compounds of general formula (I), or that at most 30 wt%, preferably at most 25 wt%, more preferably at most 20 wt%, yet more preferably at most 15 wt%, yet more preferably at most 10 wt%, yet more preferably at most 5 wt%, even more preferably at most 3 wt%, and yet even more preferably at most 1 wt% with respect to the amount of compounds of general formula (I), of further compounds, other than compounds comprising a 1, 3, 5-triazine carbamate moiety, are condensed with compounds of general formula (I).

Typically, in the seventh aspect, the present invention relates to a coating comprising a crosslinked crosslinker according to any embodiment of the first aspect, a crosslinked mixture according to any embodiment of the second aspect, or a crosslinked compound according to any embodiment of the third aspect, with the proviso that no further compounds not falling under general formula (I), are condensed with compounds of general formula (I), or that at most 30 wt%, preferably at most 25 wt%, more preferably at most 20 wt%, yet more preferably at most 15 wt%, yet more preferably at most 10 wt%, yet more preferably at most 5 wt%, even more preferably at most 3 wt%, and yet even more preferably at most 1 wt%, with respect to the amount of compounds of general formula (I), of further compounds not falling under general formula (I), are condensed with compounds of general formula (I).

In embodiments, the coating may be able to withstand at least 70, preferably at least 80, more preferably at least 100, even more preferably at least 120, even more preferably at least 150, even more preferably at least 175, yet more preferably at least 200 MEK double rubs, when measured according to the test method described in the example section.

In embodiments, the further compounds capable of condensing with compounds of general formula (I) have one or more of the following functionalities: aldehyde, hydroxyl, epoxide, carboxylic, and anhydride.

Any feature of the seventh aspect may be as correspondingly described in the first, second, third, or sixth aspect.

In an eighth aspect, the present invention relates to a method for co-crosslinking a crosslinker according to any embodiment of the first aspect, a mixture according to any embodiment of the second aspect, or a compound according to any embodiment of the third aspect, with a further compound, wherein the method comprises a step of heating up the crosslinker, the mixture or the compound and the further compound at a temperature of from 100 to 150 °C, or even from 110 to 150 °C.

The crosslinker according to any embodiment of the first aspect, the mixture according to any embodiment of the second aspect, or the compound according to any embodiment of the third aspect, can be co-crosslinked with a variety of further compounds. Examples of suitable further compounds are comonomers, oligomers, and polymers such as hydroxyl-containing monomers, oligomers, and polymers; epoxide-containing monomers, oligomers, and polymers; and carboxylic containing monomers, oligomers, and polymers.

The mixture of the crosslinker according to any embodiment of the first aspect, the mixture according to any embodiment of the second aspect, or the compound according to any embodiment of the third aspect on one hand, with the further compounds, on the other hand, can be comprised in a coating composition, and in particular in a low-VOC waterborne composition.

In an advantageous aspect, the further compound for use herein is an acrylic polyol. Exemplary suitable acrylic polyol polymers for use herein include those commercially available from allnex USA, INC under the trade designations Viacryl^{®}, Setaqua^{®} or Macrynal^{®}.

This co-crosslinking can occur with or without catalyst and typically requires heating.

The resulting coating is typically suitable for protecting surfaces.

Any feature of the eighth aspect may be as correspondingly described in the first, second, or third aspect.

### EXAMPLES

The present disclosure is further illustrated by the following examples. These examples are merely for illustrative purposes only and are not meant to be limiting on the scope of the appended claims.

### Example 1: Reaction between tris((methyl/butyl) carbamate) triazine and 2-(2-methoxyethoxy)ethanol in a molar ratio 1 : 3 of triazine to alcohol.

A 500 ml four-necked glass flask with glass stirrer, thermocouple, distillation bridge, and Liebig condenser was charged with 242 g (0.32 moles) of a 1, 3, 5-triazine containing a combination of butyl and methyl carbamate functionalities (present at 50% by weight in butanol, and commercially available from allnex under the tradename Cymel^{®} NF2000A). A distillation was carried out under reduced pressure at 100 mbar and gradual temperature increase from 70 to 150 °C until the weight of the compounds present in the glass flask was reduced to 136 g, indicating near-complete removal of the butanol solvent.

Then, the flask and its content were brought back to atmospheric pressure under a nitrogen atmosphere and cooled down to 100 °C. 118 g (0.98 moles) of 2-(2-methoxyethoxy)ethanol were then mixed with the 1, 3, 5-triazine. The temperature of the reaction mixture was gradually increased under atmospheric pressure along with the nitrogen atmosphere and kept between 120 and 130 °C for 30 minutes to facilitate exchange by 2-(2-methoxyethoxy)ethanol of the alkyl chains present on the 1, 3, 5-triazine. The vacuum was then gradually increased under a nitrogen atmosphere from 350 mbar to 80 mbar, and the temperature was then gradually increased to 150 °C. The reaction was kept at 80 mbar and 150 °C with stirring until no further condensation of distillate is observed, thereby obtaining 173g of the final product. Once all solvent was distilled, the reaction temperature was decreased to 80 °C and 48 g of water was added stepwise, thereby obtaining a 78 wt% adduct in water.

From C NMR, ~ 65 mol% (average 2 substitutions of 2-(2-methoxyethoxy)ethyl carbamate functionalities on each triazine molecule) replacement of alkylated species was achieved.

From LC-MS, a similar substitution degree was confirmed (64 mol%). Also, LC-MS allowed evaluating the proportion of tris (2-(2-Methoxyethoxy)ethylcarbamate) in the triazine to be 21 mol%.

### Example 2: Reaction between tris((methyl/butyl) carbamate) triazine and 2-(2-methoxyethoxy)ethanol in a molar ratio 1 : 4.5 of triazine to alcohol.

A 500 ml four-necked glass flask with glass stirrer, thermocouple, distillation bridge, and Liebig condenser was charged with 201 g (0.27 moles) of a 1, 3, 5-triazine containing a combination of butyl and methyl carbamate functionalities (present at 50% by weight in butanol, and commercially available from allnex under the tradename Cymel^{®} NF2000A). A distillation was carried out under reduced pressure at 100 mbar and a gradual temperature increase from 70 to 150 °C until the weight of the compounds present in the glass flask was reduced to 113 g, indicating near-complete removal of the butanol solvent.

Then, the flask and its content were brought back to atmospheric pressure under a nitrogen atmosphere and cooled down to 100 °C. 147 g (1.22 moles) 2-(2-methoxyethoxy)ethanol were then mixed with the 1, 3, 5-triazine. The temperature of the reaction mixture was gradually increased under atmospheric pressure along with the nitrogen atmosphere and kept between 120 and 130 °C for 30 minutes to facilitate exchange by 2-(2-methoxyethoxy)ethanol of the alkyl chains present on the 1, 3, 5-triazine. The vacuum was then gradually increased under a nitrogen atmosphere from 350 mbar to 80 mbar and the temperature was gradually increased to 150 °C. The reaction was kept at 80 mbar and 150 °C with stirring until no further condensation of distillate is observed, thereby obtaining 150 g of the final product. Once all solvent was distilled, the reaction temperature was decreased to 80 °C and 36 g of water was added stepwise, thereby obtaining a 81 wt% adduct in water.

From C NMR, ~ 77 mol% (average 2.3 substitutions of 2-(2-methoxyethoxy)ethyl carbamate functionalities on each triazine molecule) replacement of alkylated species was achieved.

From LC-MS, a similar substitution degree was confirmed (76 mol%). Also, LC-MS allowed to evaluate the proportion of tris (2-(2-Methoxyethoxy)ethylcarbamate) in the triazine to be 39 mol%.

### Example 3: Reaction between tris((methyl/butyl) carbamate) triazine and 2-(2-methoxyethoxy)ethanol in a molar ratio 1 : 1.5 of triazine to alcohol.

A 500 ml four-necked glass flask with glass stirrer, thermocouple, distillation bridge, and Liebig condenser was charged with 270 g (0.37 moles) of a 1, 3, 5-triazine containing a combination of butyl and methyl carbamate functionalities (present at 50% by weight in butanol, and commercially available from allnex under the tradename Cymel^{®} NF2000A). A distillation was carried out under reduced pressure at 100 mbar and a gradual temperature increase from 70 to 150 °C until the weight of the compounds present in the glass flask was reduced to 145.1 g, indicating near-complete removal of the butanol solvent.

Then, the flask and its content were brought back to atmospheric pressure under a nitrogen atmosphere and cooled down to 100 °C. 66 g (0.55 moles) of 2-(2-methoxyethoxy)ethanol were then mixed with the 1, 3, 5-triazine. The temperature of the reaction mixture was gradually increased under atmospheric pressure along with the nitrogen atmosphere and kept between 120 and 130 °C for 30 minutes to facilitate exchange by 2-(2-methoxyethoxy)ethanol of the alkyl chains present on the 1, 3, 5-triazine. The vacuum was then gradually increased under a nitrogen atmosphere from 350 mbar to 80 mbar and the temperature was gradually increased to 150 °C.

The reaction was kept at 80 mbar and 150 °C with stirring until no further condensation of distillate is observed, thereby obtaining 180 g of the final product. Once all solvent was distilled, the reaction temperature was decreased to 80 °C and 10 g of water was added stepwise. To further reduce the viscosity, 31 g of 2-butoxyethanol solvent was added, thereby obtaining a 80 wt% solid adduct in water.

From C NMR, ~ 39 mol% (average 1.17 substitutions of 2-(2-methoxyethoxy)ethyl carbamate functionalities on each triazine molecule) replacement of alkylated species was achieved.

From LC-MS, a similar substitution degree was confirmed (39 mol%). Also, LC-MS allowed evaluating the proportion of tris (2-(2-methoxyethoxy)ethyl carbamate) in the triazine to be 5 mol%.

The chemical composition of the crosslinkers of Examples 1 to 3 is summarized in Table 1 below:

**Table 1: Chemical composition of the crosslinkers of Examples 1 to 3.**

| Sample | Example 1 | | Example 2 | | Example 3 | |
|---|---|---|---|---|---|---|
| | LC-MS | C-NMR | LC-MS | C-NMR | LC-MS | C-NMR |
| Total% Methyl (mole %) | 4.0 | 3.0 | 2.5 | 2.0 | 7.5 | 6.8 |
| Total % Butyl (mole %) | 32.0 | 32.3 | 21.1 | 20.8 | 53.2 | 54.4 |
| Total % 2- (2-methoxyethoxy)ethanol (mole %) | 64.0 | 64.7 | 76.4 | 77.2 | 39.3 | 38.8 |
| Tris (2-(2-methoxyethoxy)ethyl carbamate Triazine) from LC-MS results | 21 | | 39 | | 5 | |
| Residual tris((methyl/butyl) carbamate) triazine from LC-MS results | 7 | | 2 | | 29 | |

### Example 4: Reaction between tris((methyl/butyl) carbamate) triazine and 2-butoxyethanol in a molar ratio 1 : 3 of triazine to alcohol.

A 500 ml four-necked glass flask with glass stirrer, thermocouple, distillation bridge and Liebig condenser was charged with 270 g (0.37 moles) of a 1, 3, 5-triazine containing a combination of butyl and methyl carbamate functionalities (present at 50% by weight in butanol, and commercially available from allnex under the tradename Cymel^{®} NF2000A). A distillation was carried out under reduced pressure at 100 mbar and a gradual temperature increase from 70 to 150 °C until the weight of the compounds present in the glass flask was reduced to 145.1 g, indicating near-complete evaporation of the butanol.

Then, the flask and its content were brought back to atmospheric pressure under a nitrogen atmosphere and cooled down to 100 °C. 116 g (0.98 moles) of 2-butoxyethanol were then mixed with the 1, 3, 5-triazine. The temperature of the reaction mixture was gradually increased under atmospheric pressure along with the nitrogen atmosphere and kept between 120 and 130 °C for 30 minutes to facilitate exchange by 2-Butoxyethanol of the alkyl chains present on the 1, 3, 5-triazine. The vacuum was then gradually increased under a nitrogen atmosphere from 350 mbar to 80 mbar and the temperature was gradually increased to 150 °C. The reaction was kept at 80 mbar and 150 °C with stirring until no further condensation of distillate is observed, thereby obtaining 165 g of the final product. Once all solvent was distilled, the reaction temperature was decreased to 80 °C and 9 g of water was added slowly. To further reduce the viscosity, 40 g of 2-butoxyethanol solvent was added, thereby obtaining a 77 wt% adduct in water and 2-butoxyethanol.

From C NMR, ~ 53 mol% (average 1.59 substitutions of 2-butoxyethyl carbamate functionalities on each triazine molecule) replacement of alkylated species was achieved.

### Example 5: Reaction between tris((methyl/butyl) carbamate) triazine and 2-(2-butoxyethoxy)ethanol in a molar ratio 1 : 3 of triazine to alcohol in a single distillation step.

A 500 ml four-necked glass flask with glass stirrer, thermocouple, distillation bridge, and Liebig condenser was charged with 223 g (0.30 moles) of a 1, 3, 5-triazine containing a combination of butyl and methyl carbamate functionalities (present at 50% by weight in butanol, and commercially available from allnex under the tradename Cymel^{®} NF2000A). 147 g (0.91 moles) of 2-(2-butoxyethoxy)ethanol were then mixed with the 1, 3, 5-triazine. The temperature of the reaction mixture was gradually raised to 80 °C under nitrogen atmosphere. The reaction mixture was then slowly brought to a reduced pressure of 80 mbar under a nitrogen atmosphere, while simultaneously increasing the temperature from 80 °C to 150 °C, thereby carrying out a distillation and a reaction at the same time.

The temperature was then kept at 150 °C at the pressure of 80 mbar with stirring until no further condensation of distillate is observed, thereby obtaining 189 g of the final product. The reaction temperature was then decreased to 80 °C and 29 g of water as well as 41g of 2-Butoxyethanol solvent were added stepwise, thereby obtaining a 73 wt% adduct in water and 2-butoxyethanol.

From C NMR, ~ 63 mol% (average 1.89 substitutions of 2-(2-butoxyethoxy)ethyl carbamate functionalities on each triazine molecule) replacement of alkylated species was achieved.

From LC-MS, a similar substitution degree was confirmed (61 mol%). Also, LC-MS allowed evaluating the proportion of tris (2-(2-butoxyethoxy)ethyl carbamate) in the triazine to be 30 mol%.

### Example 6: Reaction between tris((methyl/butyl) carbamate) triazine and dipropylene glycol monomethyl ether in a molar ratio 1 : 3 of triazine to alcohol in a single distillation step.

A 500 ml four-necked glass flask with glass stirrer, thermocouple, distillation bridge, and Liebig condenser was charged with 221 g (0.30 moles) of a 1, 3, 5-triazine containing a combination of butyl and methyl carbamate functionalities (present at 50% by weight in butanol, and commercially available from allnex under the tradename Cymel^{®} NF2000A). 132.8 g (0.90 moles) of a dipropylene glycol monomethyl ether which is commercially available under the trade designation DOWANOL^{™} DPM Glycol Ether were then mixed with the 1, 3, 5-triazine. The temperature of the reaction mixture was gradually raised to 80 °C under nitrogen atmosphere. The reaction mixture was then slowly brought to a reduced pressure of 80 mbar under a nitrogen atmosphere, while simultaneously increasing the temperature from 80°C to 150°C, thereby carrying out a distillation and a reaction at the same time. The temperature was then kept at 150 °C at the pressure of 80 mbar with stirring until no further condensation of distillate is observed, thereby obtaining 147 g of the final product. Once all solvent was distilled, the reaction temperature was decreased to 80 °C and 9 g of water was added. To further reduce the viscosity, 48.5 g of 2-butoxyethanol was added stepwise, thereby obtaining a 72 wt% adduct in water and 2-butoxyethanol.

From C NMR, ~ 40 mol% (average 1.21 substitutions of dipropylene glycol monomethyl ether carbamate functionalities on each triazine molecule) replacement of alkylated species was achieved.

### Example 7: Waterborne compositions.

Waterborne compositions could be obtained from all the crosslinkers of examples 1 to 6. Furthermore, all the crosslinkers of the present invention show excellent water dispersibility properties. No additional organic solvent was needed at all for the crosslinkers of examples 1 and 2 to achieve good miscibility in water. Some amount of organic solvent was beneficial to obtain good miscibility in water for the crosslinkers of examples 3 to 6.

### Example 8: Self-crosslinking performance.

The evaluation of the self-crosslinking properties of the crosslinkers of the present invention and of Cymel^{®} NF2000A (as a comparative triazine) was carried out by forming a coating on a metal panel, followed by heating up the coating at 140 °C for 30 min. All obtained films were clear. No catalyst was used for the self-crosslinking study.

Solvent resistance (Methyl Ethyl Ketone double rubs on metal) was assessed by pressing a cotton rag saturated with Methyl Ethyl Ketone, respectively, with a backward and forward motion on the coated surface; one double rub is equal to a backward and forward stroke on the coated surface. The reported number is the number of double rubs required to break through the coating. A high solvent resistance is advantageous to ensure a good protection of the coating and the substrate against any household or industrial product spillage.

From these studies, it was concluded that the comparative tris((methyl/butyl) carbamate) triazine (Cymel^{®} NF2000A) does not self-crosslink while the crosslinkers of the present invention do. The crosslinkers obtained in all the examples according to the invention displayed excellent self-crosslinking properties as demonstrated by their ability to resist to at least 75, at least 100, at least 150, or even at least 200 methyl ethyl ketone double rubs. All other examples of crosslinkers according to the present invention showed better self-crosslinking properties with higher methyl ethyl ketone double rub resistance than the Cymel ^{®} NF2000A control, which failed before 40 methyl ethyl ketone double rubs.

The results are summarized in the Table 2 below:

**Table 2: Self-crosslinking performance.**

| Sample | MEK double rubs (140 °C) |
|---|---|
| Example 1 | 200 |
| Example 2 | 200 |
| Example 3 | 75 |
| Example 4 | 175 |
| Example 5 | 150 |
| Example 6 | 200 |
| Cymel^{®} NF2000A control | 39 |

### Example 9: Co-crosslinking in low VOC formulations.

Although the comparative tris((methyl/butyl) carbamate) triazine Cymel^{®} NF2000A provides a lot of beneficial properties, it is however not water-soluble. It can be used in water-reducible resins and with waterborne dispersions but this requires prior blending with the resin to be crosslinked, followed by adding a water compatible organic solvent. This procedure, however, does not always allow obtaining stable water-based formulations. Furthermore, the resulting formulations are relatively high in VOC (Volatile Organic Compounds).

By contrast, the crosslinkers of the present invention are typically waterborne or do not require much organic solvent to form a clear and stable composition. When waterborne, they are typically obtained with less than 2wt% of organic solvent. Otherwise, no more than 15 wt% organic solvent is sufficient to make it compatible with water.

As these crosslinking agents do not contribute much to the volatile organic component (VOC) in the coating formulation, formulations prepared with the crosslinkers of the present invention and typical water-based binder, in particular waterborne acrylic resins, show a significant reduction in VOC as compared to formulations prepared with Cymel^{®} NF2000A and a binder.

The evaluation of the co-crosslinking properties of Cymel^{®} NF2000A and the crosslinkers of the present invention along with a waterborne acrylic resin (commercial available from allnex under the trade designation Viacryl^{®} 6276) was carried out by forming a coating on a metal panel, followed by heating the coating at a temperature of from 115 °C to 140 °C for 30 min. All obtained films were clear. No catalyst was used for the co-crosslinking study.

Low VOC formulations could be obtained, although it is notable that the polymeric binder Viacryl^{®} 6276 comprises 3.2 wt% of organic solvent which necessarily increases the overall VOC values. The VOC results in g/L as well as methyl ethyl ketone double rubs results are presented in Table 3 below.

**Table 3: Co-crosslinking performance and VOC values.**

| Sample | Binder Viacryl 6276 / Sample weight ratio | T° (MEK rubs) |
|---|---|---|
| Example 1 | 79/21 | 120 °C (200 rubs) |
| Example 2 | 79/21 | 140 °C (200 rubs) |
| Example 3 | 79/21 | 120 °C (200 rubs) |
| Example 4 | 79/21 | 130 °C (175 rubs) |
| Example 5 | 76/24 | 140 °C (200 rubs) |
| Example 6 | 77/23 | 120 °C (200 rubs) |
| Cymel^{®} NF2000A control | 84/16 | 120 °C (200 rubs) |

As is readily observed in Table 3, the co-crosslinking performances of the crosslinkers according to the present invention with the exemplified waterborne acrylic resin Viacryl^{®} 6276 are at par with that obtained with the Cymel^{®} NF2000A control.

These co-crosslinking experiments have been repeated on glass and paper panels instead of metal panels. On these substrates too, good methyl ethyl ketone double rubs performances were obtained.

## Claims

1. A crosslinker obtainable by reacting one or more compounds of general formula (II) 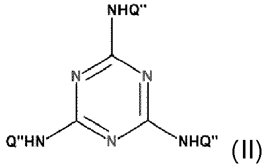 with one or more compounds of general formula (III)
HO-[X -]ᵢR¹ (III),
wherein each Q" is a radical of formula COOR²,
wherein each R² is independently selected from the group consisting of C₁-C₃₀ hydrocarbyls,
wherein each X is independently selected from the group consisting of CH₂-CH₂-O, CH₂-CH(CH₃)-O, CH(CH₃)-CH₂-O-, CH₂-C(CH₃)₂-O-, CH₂-CHVin-O, CHVin-CH₂-O, CH₂-CHPh-O-, and CHPh-CH₂-O, in which Ph is phenyl and Vin is vinyl,
wherein each i is independently selected from integers between 1 and 50, wherein each R¹ is independently selected from the group consisting of C₁-C₃₀ hydrocarbyls,
wherein the crosslinker comprises a mixture of compounds of general formula (I) 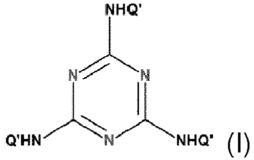
wherein Q' is a radical of formula COO-[X-]ᵢR¹,
wherein each i is independently selected from integers between 1 and 50 for 35 mol% or higher of Q' within the mixture, and i is equal to 0 for the remaining Q' within the mixture,
wherein each X is independently selected from the group consisting of CH₂-CH₂-O, CH₂-CH(CH₃)-O, CH(CH₃)-CH₂-O-, CH₂-C(CH₃)₂-O-, CH₂-CHVin-O, CHVin-CH₂-O, CH₂-CHPh-O-, and CHPh-CH₂-O, in which Ph is phenyl and Vin is vinyl, and
wherein each R¹ is independently selected from the group consisting of C₁-C₃₀ hydrocarbyls.

2. The crosslinker according to claim 1, wherein at least 70 mol% of the compounds of the mixture have at least one Q' radical having an i different from 0.

3. The crosslinker according to claim 1 or claim 2, wherein each i is independently selected from integers between 1 and 50 for from 59 to 77 mol% of Q' within the mixture, and i is equal to 0 for the remaining Q' within the mixture.

4. The crosslinker according to claim 3, wherein i equals 2 for from 59 to 77 mol% of Q' within the mixture, and i is equal to 0 for the remaining Q' within the mixture.

5. The crosslinker according to any one of the preceding claims, wherein each X is independently selected from the group consisting of -CH₂-CH₂-O, and -CH₂-CH(CH₃)-O.

6. The crosslinker according to any one of the preceding claims, wherein R¹ is independently selected from the group consisting of C₁-C₄ hydrocarbyls.

7. The crosslinker according to any one of the preceding claims, wherein at least 5 mol% of the compounds of formula (I) in the mixture have an i selected from integers between 1 and 50 for all its three Q' groups.

8. A waterborne composition comprising a crosslinker according to any one of claims 1 to 7.

9. A method for forming a crosslinker according to any one of claims 1 to 7, comprising the steps of reacting one or more compounds of general formula (II) 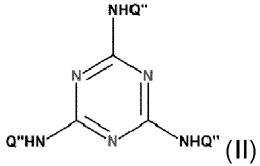 with one or more compounds of general formula (III)
HO-[X-]ᵢR¹ (III),
in absence of a catalyst,
wherein Q" is a radical of formula COOR²,
wherein each R² is independently selected from the group consisting of C₁-C₃₀ hydrocarbyls,
wherein each X is independently selected from the group consisting of CH₂-CH₂-O , CH₂-CH(CH₃)-O, CH(CH₃)-CH₂-O-, CH₂-C(CH₃)₂-O-, CH₂-CHVin-O , CHVin-CH₂-O , CH₂-CHPh-O-, and CHPh-CH₂-O, in which Ph is phenyl and Vin is vinyl,
wherein each i is independently selected from integers between 1 and 50, wherein each R¹ is independently selected from the group consisting of C₁-C₃₀ hydrocarbyls,

10. A method for forming a coating comprising reacting a crosslinker according to any one of claims 1 to 7 with itself, with the proviso that the reaction is performed in presence of at most 30 wt% with respect to the amount of compounds of general formula (I), of further compounds, other than compounds comprising a 1, 3, 5-triazine carbamate moiety, capable of condensing with compounds of general formula (I).

11. The method according to claim 10, wherein the further compounds capable of condensing with compounds of general formula (I) have one or more of the following functionalities: aldehyde, hydroxyl, epoxide, carboxylic, and anhydride.

12. The method according to claim 10 or claim 11, wherein the reaction step comprises subjecting the crosslinker to a temperature of at least 120 °C.

13. A coating comprising a crosslinked crosslinker according to any one of claims 1 to 7 with the proviso that no further compounds, other than compounds comprising a 1, 3, 5-triazine carbamate moiety, are condensed with compounds of general formula (I), or that at most 30 wt% with respect to the amount of compounds of general formula (I), of further compounds, other than further compounds comprising a 1, 3, 5-triazine carbamate moiety, are condensed with compounds of general formula (I).

14. A method for co-crosslinking a crosslinker according to any one of claims 1 to 7 with a further compound, comprising a step of heating up the crosslinker and the further compound at a temperature of from 100 to 150 °C.

15. A compound of general formula (I) 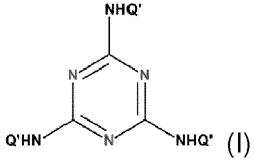 selected from the list consisting of compounds a) wherein one of the Q' groups is a radical of formula COOCH₂CH₂CH₂CH₃, and wherein two of the Q' groups are radicals of formula COO-[CH₂-CH₂-O-]₂CH₃, b) wherein one of the Q' groups is a radical of formula COOCH₂CH₂CH₂CH₃, and wherein two of the Q' groups are radicals of formula COO-[CH₂-CH₂-O-]₂CH₂CH₂CH₂CH₃, c) wherein one of the Q' groups is a radical of formula COOCH₂CH₂CH₂CH₃, and wherein two of the Q' groups are radicals of formula COO-[CH₂-CH(CH₃)-O-]₂CH₃, and d) wherein one of the Q' groups is a radical of formula COOCH₂CH₂CH₂CH₃, and wherein two of the Q' groups are radicals of formula COOCH₂CH₂OCH₂CH₂CH₂CH₃.
